# EUROPÄISCHE PATENTSCHRIFT

(11) **EP 2 526 081 B1**
(45) Veröffentlichungstag und Bekanntmachung des Hinweises auf die Patenterteilung: **01.07.2015**
(21) Anmeldenummer: 11700418.4
(22) Anmeldetag: 14.01.2011
(51) Int. Cl.: C07D 295/192, C07C 69/612, C07C 67/343, C07C 45/68, C07C 69/734, C07C 69/76, C07C 69/738, C07C 69/616

(54) **VERFAHREN ZUR HERSTELLUNG VON ARYL- UND HETEROARYLESSIGSÄURE-DERIVATEN**
METHOD FOR PRODUCING ARYL AND HETEROARYLACETIC ACID DERIVATIVES
PROCÉDÉ DE PRODUCTION DE DÉRIVÉS D'ACIDES ARYL- ET HÉTÉROARYLACÉTIQUE

(30) Priorität: 19.01.2010 US 296081 P; 19.01.2010 EP 10151081
(43) Veröffentlichungstag der Anmeldung: 28.11.2012
(73) Patentinhaber: Bayer Intellectual Property GmbH, 40789 Monheim (DE)
(72) Erfinder: HIMMLER, Thomas, 51519 Odenthal (DE); GOOßEN, Lukas, J., 67663 Kaiserslautern (DE); ZIMMERMANN, Bettina, 66978 Clausen (DE)
(86) Internationale Anmeldenummer: PCT/EP2011/050456
(87) Internationale Veröffentlichungsnummer: WO 2011/089072

(56) Entgegenhaltungen:
- E. PAETZOLD, G. OEHME: "Efficient two-phase Suzuki reaction catalyzed by palladium complexes with water-soluble phosphine ligands and detergents as phase transfer reagents", JOURNAL OF MOLECULAR CATALYSIS A: CHEMICAL, Bd. 152, 2000, Seiten 69-76, XP002588492,
- PIERRE GENET J ET AL: "Recent developments of palladium(0) catalyzed reactions in aqueous medium", JOURNAL OF ORGANOMETALLIC CHEMISTRY, ELSEVIER-SEQUOIA S.A. LAUSANNE, CH LNKD- DOI:10.1016/S0022-328X(98)01088-2, Bd. 576, Nr. 1-2, 15. März 1999 (1999-03-15), Seiten 305-317, XP004166293, ISSN: 0022-328X
- LU T-Y ET AL: "Palladium-catalyzed cross-coupling reaction of aryldioxaborolane with 2-bromo-N,N-dimethylacetamide", TETRAHEDRON LETTERS, ELSEVIER, AMSTERDAM, NL LNKD- DOI:10.1016/S0040-4039(03)00066-2, Bd. 44, Nr. 8, 17. Februar 2003 (2003-02-17), Seiten 1587-1590, XP004405277, ISSN: 0040-4039
- GOOSSEN L J: "PD-CATALYZED SYNTHESIS OF ARYLACETIC ACID DERIVATIVES FROM BORONIC ACIDS", CHEMICAL COMMUNICATIONS - CHEMCOM, ROYAL SOCIETY OF CHEMISTRY, GB LNKD- DOI:10.1039/B100834J, Nr. 7, 1. Januar 2001 (2001-01-01), Seite 669/670, XP001088567, ISSN: 1359-7345

## Beschreibung

Die Erfindung betrifft ein Verfahren zur Herstellung von Aryl- und Heteroarylessigsäuren und ihrer Derivate durch Umsetzung von Aryl- oder Heteroarylboronsäurederivaten mit α-Halogenessigsäuren bzw. ihrer Derivate in Gegenwart eines Palladiumkatalysators, einer Base und eines Phasentransferkatalysators. Dieses Verfahren ermöglicht die Darstellung einer Vielzahl funktionalisierter Aryl- und Heteroarylessigsäuren und ihrer Derivate. Es ist darüber hinaus auch anwendbar zur Herstellung anderer α-Arylcarbonylverbindungen.

Üblicherweise werden Phenylessigsäure-Derivate in mehrstufigen Synthesen hergestellt, die meist eine geringe Gruppentoleranz aufweisen. Die Herstellung kann beispielsweise ausgehend von Acetophenonen durch eine Willgerodt-Kindler-Reaktion erfolgen (siehe beispielsweise H.E. Zaugg et al., J. Amer. Chem. Soc. 70 (1948) 3224-8). Bei dieser Methode fallen jedoch große Mengen schwefelhaltiger Abfälle an. Außerdem können stark geruchsbelästigende leichtflüchtige Schwefelverbindungen auftreten.

Eine weitere Methode zur Herstellung von Arylessigsäuren geht von Benzylbromiden oder -chloriden aus. Man stellt daraus z.B. mit Natriumcyanid die entsprechenden Nitrile her, welche anschließend verseift werden. Die benötigten Benzylbromide oder -chloride können beispielsweise durch Brom- oder Chlormethylierung der entsprechenden Aromaten erhalten werden. Hierbei ist allerdings nachteilig, dass das Auftreten stark kanzerogener Verbindungen wie Bis(chlormethyl)ether oder Bis(brommethyl)ether nicht ausgeschlossen werden kann, so dass technisch ein hoher Sicherheitsaufwand betrieben werden muss. Außerdem führt die Halogenmethylierung von substituierten Aromaten in vielen Fällen zu Isomerengemischen.

Die Carbonylierung von Benzylhalogeniden in Gegenwart von Alkoholen liefert ebenfalls Phenylessigsäureester. Die bereits genannte begrenzte Verfügbarkeit von Benzylhalogeniden und die Notwendigkeit, toxisches CO-Gas einzusetzen, gegebenenfalls auch unter erhöhtem Druck, sind weitere Nachteile dieses Verfahrens.

Es ist auch bereits bekannt geworden, α-Chlor-acetophenone zu ketalisieren und die Ketale dann einer Umlagerungsreaktion zu unterwerfen (C.Giordano et al., Angew. Chem. 96 (1984) 413-9). Die α-Chlor-acetophenone erhält man entweder durch Chlorierung von Acetophenonen oder direkt durch eine Friedel-Crafts-Acylierung des betreffenden Aromaten mit Chloracetylchlorid. Damit ergibt sich wieder der Nachteil, dass die Friedel-Crafts-Acylierungen an substituierten Aromaten häufig unselektiv verlaufen.

Eine weitere bekannte Methode zur Herstellung von Phenylessigsäuren besteht darin, ein entsprechendes Anilin im ersten Schritt zu diazotieren, die erhaltene Diazoniumverbindung im zweiten Schritt mit Vinylidenchlorid umzusetzen, und die so erhaltene Trichlor- oder Brom-dichlorethylverbindung dann im dritten Schritt mit Wasser oder Alkoholen zur Arylessigsäure bzw. deren Estern umzusetzen (siehe beispielsweise V.M.Naidan und A.V.Dombrovskii, Zhurnal Obshchei Khimii 34 (1984)1469-73; EP-A-835243). Diese Reaktion liefert in der Regel jedoch nur mit solchen Anilinen gute Ausbeuten, die elektronenziehende Reste am Aromaten tragen und in denen die Aminogruppe nicht sterisch blockiert ist.

Weiterhin bekannt ist die Umsetzung von Brombenzolen mit Chloressigsäurederivaten in Gegenwart von stöchiometrischen Mengen Silber oder Kupfer bei 180 - 200 °C. Nachteilig an diesen Verfahren ist die hohe Temperatur, die eine Anwendung bei temperaturempfindlichen Verbindungen ausschließt, die geringe Ausbeute und die Anwendung stöchiometrischer Mengen teurer und schwer wieder aufzuarbeitender Metalle.

Die Umsetzung von Aryl-Grignardverbindungen mit α-Halogenessigsäure-Derivaten führt ebenfalls zu Phenylessigsäurederivaten. Nachteilig ist allerdings die durch die Verwendung von schwer zu handhabenden, hochreaktiven Grignardverbindungen äußerst eingeschränkte Toleranz funktioneller Gruppen.

Ebenso ist bekannt geworden Arylessigsäurederivate dadurch herzustellen, dass man Arylhalogenide mit Dialkylmalonaten unter gleichzeitiger De-alkoxycarbonylierung umsetzt (Tetrahedron Lett. 2004, 45, 5823-5). Dies hat jedoch den Nachteil, dass als Base teures Cesiumcarbonat benötigt wird.

Alternativ zu den genannten Verfahren wurden auch Kreuzkupplungen von Arylhalogeniden mit Reformatsky-Reagenzien, Zinn-, Kupfer- und anderen Enolaten oder Ketenacetalen beschrieben (siehe z. B. J. Am. Chem. Soc. 1959, 81, 1627-1630*;* J. Organomet. Chem. 1979, 177, 273-281; Synth. Comm. 1987, 17, 1389-1402; Bull. Chem. Soc. Jpn. 1985, 58, 3383-3384; J. Org. Chem. 1993, 58, 7606-7607; J. Chem. Soc. Perkin 1 1993, 2433-2440; J. Am. Chem. Soc. 1975, 97, 2507-2517; J. Am. Chem. Soc. 1977, 99, 4833-4835; J. Am. Chem. Soc. 1999, 121, 1473-78; J. Org. Chem. 1991, 56, 261-263, Heterocycles 1993, 36, 2509-2512, Tetrahedron Lett. 1998, 39, 8807-8810. Übersichten zu solchen Reaktionen finden sich in: Chem. Rev. 2010, 110, 1082-1146 und Angew. Chem. 2010, 122, 686-718). Diese Verfahren sind allerdings in ihrer Anwendbarkeit limitiert. So sind Reformatzky-Reagenzien und Ketenacetale aufwendig in der Darstellung und Handhabung. Die Verwendung von Zinnverbindungen ist aus toxikologischen Gründen nachteilig und der Einsatz von stöchiometrischen Mengen Kupfer verursacht erhebliche Kosten bei der Entsorgung. Die Verwendung von Enolaten ist in der Regel nur möglich, wenn keine weiteren enolisierbaren Gruppen im Molekül vorhanden sind. Beispielsweise sind Ketone daher als Substrate für derartige Verfahren ausgeschlossen. Einige elektrochemische Prozesse sind ebenfalls bekannt (Synthesis 1990, 369-381*;* J. Org. Chem. 1996, 61, 1748-1755), allerdings sind diese Verfahren aufgrund der aufwendigen Reaktionsführung und der geringen Raum-Zeit-Ausbeuten nachteilig.

Ebenfalls bereits bekannt ist eine Methode zur Herstellung von Phenylessigsäure-Derivaten durch eine Palladium-katalysierte Kupplungsreaktion zwischen breit zugänglichen, leicht handhabbaren und stabilen Arylboronsäuren und Ethylbromacetat (L.J.Gooßen, Chem.Commun. 2001, 660-70*;* DE-A-10111262). Allerdings konnte dieses Verfahren bisher nicht zur Herstellung von sterisch anspruchsvollen, beispielsweise 2,6-disubstituierten Phenylessigsäure-Derivaten verwendet werden. In Chem. Commun. 2001, 660-70 wird zwar angegeben, dass auch sterisch gehinderte Arylboronsäuren unter den dort beschriebenen Bedingungen gut umgesetzt werden können. Die Beispiele enthalten jedoch nur die 2-Tolylboronsäure als sterisch gehindertes Substrat. Stärker sterisch eingeschränkte Arylboronsäuren wie z.B. 2,6-Dialkylphenyl-boronsäuren werden nicht beschrieben. Eigene Versuche (siehe Vergleichsbeispiel 1) belegen, dass die oben zitierte Methode in solchen Fällen nur unbefriedigende Ausbeuten an Arylessigsäurederivaten liefert.

Alle bisher bekannt gewordenen Methoden zur Herstellung von sterisch anspruchsvoll substituierten Phenylessigsäure-Derivaten weisen demnach z.T. erhebliche Mängel und Nachteile auf, die ihre Anwendung erschweren. Da allgemein Phenylessigsäuren, und unter ihnen gerade auch sterisch anspruchsvoll substituierte, wichtige Vorprodukte beispielsweise für Wirkstoffe im Pflanzenschutz sind, besteht Bedarf an einer technisch einfachen und hocheffizienten Methode zur Herstellung solcher Verbindungen.

Überraschenderweise wurde nun ein Verfahren zur Herstellung von Aryl- und Heteroarylessigsäuren und ihrer Derivate aus Aryl- und Heteroarylboronsäurederivaten und α-Halo-essigsäuren und ihren Derivaten gefunden, welches dadurch gekennzeichnet ist, dass die Umsetzung in Gegenwart von einem Palladiumkatalysator, einem Phosphin, einer anorganischen Base und einem Phasentransferkatalysator durchgeführt wird.

Die Entdeckung, dass der Zusatz eines Phasentransferkatalysators die Selektivität der Umsetzung positiv beeinflusst, war nicht vorhersehbar und macht die Entdeckung dieses Verfahrens besonders überraschend.

Durch den Einsatz des Phasentransferkatalysators ist es erstmals möglich, die Selektivität signifikant zu Gunsten des gewünschten Produktes zu verschieben. Insbesondere wird die Bildung von Arenen über Protodeborylierung unterdrückt. Es entstehen nur noch geringe Anteile an unerwünschten Nebenprodukten.

Außerdem bewirkt der Zusatz des Phasentransferkatalysators, dass die für eine möglichst vollständige Umsetzung notwendige Menge an Palladiumkatalysator stark gesenkt werden kann. Dies macht das Verfahren deutlich wirtschaftlicher als das nach dem Stand der Technik bekannte Verfahren

Das erfindungsgemäße Verfahren ist nicht auf sterisch anspruchsvoll substituierte Arylboronsäuren beschränkt. Auch andersartig substituierte Arylboronsäuren können unter den erfindungsgemäßen Bedingungen in besseren Ausbeuten umgesetzt werden.

Das erfindungsgemäße Verfahren zur Herstellung von Aryl- und Heteroarylcarbonylverbindungen ist dadurch gekennzeichnet, dass man Aryl- oder Heteroarylboronsäuren der Formel (I) 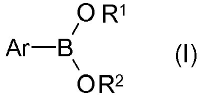 in welcher
R¹ für Wasserstoff oder C₁-C₈-Alkyl steht,
R² für Wasserstoff oder C₁-C₈-Alkyl steht, oder
R¹ und R² gemeinsam mit den Atomen, an die sie gebunden sind für einen gesättigten oder ungesättigten, substituierten oder unsubstituierten Cyclus stehen,
Ar für die Gruppe 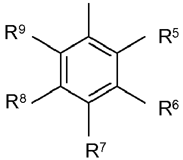 steht, wobei
R⁵, R⁶, R⁷, R⁸ und R⁹ unabhängig voneinander gleich oder verschieden für Wasserstoff, Halogen, für gegebenenfalls durch Halogen substituiertes C₁-C₆-Alkyl, C₁-C₆-Alkoxy, Phenyl, -CO-C₁-C₃-Alkyl, -COO-C₁-C₆-Alkyl oder -COO-C₆-C₁₀-Aryl stehen,
der Rest Ar kann darüber hinaus auch für einen heteroaromatischen Rest wie 2-Pyridyl, 3-Pyridyl, 4-Pyridyl, 2-Furyl-, 3-Furyl, 2-Thienyl, oder 3-Thienyl stehen oder
der Rest Ar kann auch für 1- oder 2-Naphthyl stehen,
mit α-Halogenmethyl-carbonylverbindungen der Formel (II) 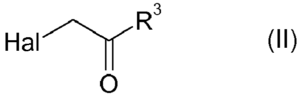 in welcher
Hal für Halogen steht,
R³ für Hydroxy, für jeweils gegebenenfalls substituiertes C₁-C₈-Alkyl, C₁-C₈-Alkoxy, Phenyl, Aryl, Phenoxy oder Aryloxy oder für NR⁴R^{4'} steht,
wobei R⁴ und R^{4'} unabhängig voneinander gleich oder verschieden für Wasserstoff, C₁-C₄-Alkyl oder für gegebenenfalls durch C₁-C₃-Alkyl, welches durch Fluor oder Chlor substituiert sein kann, durch Nitro, Cyano oder Di-C₁-C₃-Alkylamino substituiertes Phenyl stehen, oder gemeinsam mit dem Stickstoffatom an das sie gebunden sind für einen gesättigten oder ungesättigten, substituierten oder unsubstituierten Cyclus stehen,
in Gegenwart eines Palladiumkatalysators, eines Phosphin-Liganden, einer anorganischen Base, und eines Phasentransferkatalysators gegebenenfalls unter Verwendung eines organischen Lösungsmittels zu α-Arylmethyl-carbonylverbindungen der Formel (III) 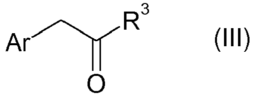 in welcher Ar und R³ die oben angegebenen Bedeutungen haben
umsetzt.

Diese Umsetzung wird durch die folgende Reaktionsgleichung veranschaulicht:

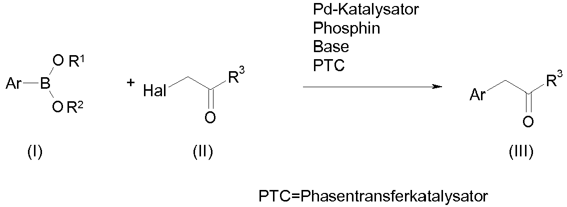

Bevorzugte Substituenten bzw. Bereiche der in der oben und nachstehend erwähnten Formeln aufgeführten Reste werden im Folgenden erläutert:
R¹ steht bevorzugt für Wasserstoff oder C₁-C₄-Alkyl,
R² steht bevorzugt für Wasserstoff oder C₁-C₄-Alkyl, oder
R¹ und R² stehen bevorzugt gemeinsam mit den Atomen, an die sie gebunden sind für gegebenenfalls durch C₁-C₄-Alkyl oder Aryl (insbesondere Phenyl) substituiertes C₂-C₃-Alkandiyl,
R³ steht bevorzugt für Hydroxy, gegebenenfalls durch Fluor substituiertes C₁-C₄-Alkyl, C₁-C₄-Alkoxy, für jeweils gegebenenfalls substituiertes Phenyl, Phenoxy oder für NR⁴R^{4'},
wobei R⁴ und R^{4'} bevorzugt unabhängig voneinander gleich oder verschieden für Wasserstoff, Methyl, Ethyl, i-Propyl, n-Propyl oder für gegebenenfalls durch Methyl, Ethyl, i-Propyl, n-Propyl, CF₃, C₂F₅, C₃F₇, Nitro, Cyano, N (Methyl)₂, N(Ethyl)₂, N(n-Propyl)₂, N(i-Propyl)₂ substituiertes Phenyl stehen, oder gemeinsam mit dem Stickstoffatom an das sie gebunden sind für einen gesättigten oder ungesättigten, substituierten oder unsubstituierten 5- oder 6-Cyclus stehen,

Ar steht bevorzugt für 1- oder 2-Naphthyl oder für die Gruppe 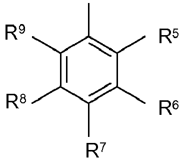 wobei
R⁵, R⁶, R⁷, R⁸ und R⁹ stehen bevorzugt unabhängig voneinander gleich oder verschieden für Wasserstoff, Fluor, Chlor, für gegebenenfalls durch Fluor substituiertes C₁-C₄-Alkyl, C₁-C₄-Alkoxy, Phenyl, -CO-C₁-C₃-Alkyl, -COO-C₁-C₄-Alkyl oder -COO-C₆-C₈-Aryl stehen,
Hal steht bevorzugt für Fluor, Chlor, Brom oder Iod.
R¹ steht besonders bevorzugt für Wasserstoff, Methyl, Ethyl, i-Propyl oder n-Propyl,
R² steht besonders bevorzugt für Wasserstoff, Methyl, Ethyl, i-Propyl oder n-Propyl, oder
R¹ und R² stehen besonders bevorzugt gemeinsam mit den Atomen, an die sie gebunden sind für gegebenenfalls einfach bis vierfach durch Methyl substituiertes C₂-Alkandiyl, für gegebenenfalls einfach bis sechsfach durch Methyl substituiertes C₃-Alkandiyl (hervorgehoben sind -CH₂C(CH₃)₂CH₂-, -C(CH₃)₂C(CH₃)₂-),
R³ steht besonders bevorzugt für Methyl, Ethyl, i-Propyl, n-Propyl, CF₃, C₂F₅, C₃F₇, Methoxy, Ethoxy, i-Propoxy, n-Propoxy oder tert.-Butoxy, für jeweils gegebenenfalls substituiertes Phenyl oder für NR⁴R^{4'},
wobei R⁴ und R^{4'} besonders bevorzugt unabhängig voneinander gleich oder verschieden für Wasserstoff, Methyl, Ethyl, i-Propyl, n-Propyl oder gemeinsam mit dem Stickstoffatom an das sie gebunden sind für einen gesättigten, unsubstituierten 5- oder 6-Cyclus stehen,
Ar steht besonders bevorzugt für 1-Naphthyl oder für die Gruppe 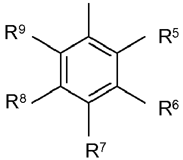 wobei
R⁵, R⁶, R⁷, R⁸ und R⁹ stehen besonders bevorzug unabhängig voneinander gleich oder verschieden für Wasserstoff, Fluor, Chlor, Methyl, Ethyl, , i-Propyl, n-Propyl CF₃, C₂F₅, C₃F₇, Methoxy, Ethoxy, Phenyl, -CO-Methyl, -CO-Ethyl, -COO-Methyl, -COO-Ethyl oder -COO-Phenyl stehen,
Hal steht besonders bevorzugt für Chlor, Brom oder Iod.
R¹ steht ganz besonders bevorzug für Wasserstoff,
R² steht ganz besonders bevorzug für Wasserstoff,
R³ steht ganz besonders bevorzugt für Methoxy, Ethoxy, tert.-Butoxy, Phenyl oder für NR⁴R^{4'},
wobei R⁴ und R^{4'} gemeinsam mit dem Stickstoffatom, an das sie gebunden sind für einen gesättigten, unsubstituierten 6-Cyclus stehen,

Ar steht ganz besonders bevorzug für 1-Naphthyl, Phenyl, 2,6-Dimethylphenyl, 2,4,6-Trimethylphenyl, 4-Acetylphenyl, 4-Chlor-2,6-dimethylphenyl, 2,6-Diethyl-4-methylphenyl, 4-Methoxyphenyl, 4-Ethoxycarbonylphenyl,

Hal steht ganz besonders bevorzugt für Brom.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Zwischenprodukte entsprechend.

Die Boronsäuren der Formel (I) sind prinzipiell bekannt oder können nach bekannten Methoden hergestellt werden, beispielsweise aus den entsprechenden Bromaromaten, Magnesiummetall und B orsäure-trimethylester.

Wahlweise können die Boronsäuren auch in situ durch Umsetzung entsprechender Arylhalogenide bzw. Heteroarylhalogenide entweder mit einer Diborverbindung oder einem Boran in Gegenwart eines Palladiumkatalysators gemäß dem Stand der Technik erzeugt werden.

Die Verbindungen der Formel (II) sind prinzipiell bekannt oder können nach bekannten Methoden hergestellt werden.

Als Basen werden im erfindungsgemäßen Verfahren anorganische Basen wie Alkali- oder Erdalkalihydroxide, -carbonate, -bicarbonate, -oxide, -phosphate, -hydrogenphosphate, -fluoride oder -hydrogenfluoride eingesetzt. Vorzugsweise werden Alkali- und Erdalkaliphosphate, -carbonate oder -fluoride verwendet und besonders bevorzugt werden Kaliumfluorid, Kaliumcarbonat und Kaliumphosphat verwendet. Hervorgehoben ist Kaliumfluorid.

Im erfindungsgemäßen Verfahren werden 1 bis 10 Äquivalente der jeweiligen Base eingesetzt. Vorzugsweise werden 2-7 Äquivalente der Base eingesetzt.

Als Palladium-Katalysatoren werden im erfindungsgemäßen Verfahren Palladium(II)-Salze wie etwa Palladiumchlorid, -bromid, -iodid, -acetat, -acetylacetonat, die wahlweise durch weitere Liganden wie z. B. Alkylnitrile stabilisiert sein können, bzw. Pd(0)-Spezies wie Palladium auf Aktivkohle, Pd(PPh₃)₄, Bis(dibenzylidenaceton)palladium oder Tris(dibenzylidenaceton)dipalladium eingesetzt. Bevorzugt sind Bis(dibenzylidenaceton)palladium, Tris(dibenzylidenaceton)dipalladium, Palladiumchlorid, Palladiumbromid und Palladiumacetat; hervorgehoben ist Bis(dibenzylidenaceton)palladium.

Die Menge an im erfindungsgemäßen Verfahren eingesetzten Palladium-Katalysator beträgt 0,001 bis 5 Molprozent, bezogen auf eingesetzte Arylboronsäure. Bevorzugt werden 0,005 bis 3 Molprozent eingesetzt; besonders bevorzugt sind 0,01 bis 1 Molprozent.

Als Phosphinliganden werden im erfindungsgemäßen Verfahren Liganden PR¹⁰R¹¹R¹² eingesetzt, wobei die Reste R¹⁰, R¹¹ und R¹² für Wasserstoff, lineares und verzweigtes C₁-C₈-Alkyl, Vinyl-, Aryl-, oder Heteroaryl aus der Reihe Pyridin, Pyrimidin, Pyrrol, Thiophen oder Furan stehen, die ihrerseits mit weiteren Substituenten aus der Reihe lineares und verzweigtes C₁-C₈-Alkyl oder C₆-C₁₀-Aryl, lineares und verzweigtes C₁-C₈-Alkyloxy oder C₁-C₁₀-Aryloxy, halogeniertes lineares und verzweigtes C₁-C₈-Alkyl oder halogeniertes C₆-C₁₀-Aryl, lineares und verzweigtes C₁-C₈-Alkyl oder C₆-C₁₀-Aryloxycarbonyl, lineares und verzweigtes C₁-C₈-Alkylamino, lineares und verzweigtes C₁-C₈-Dialkylamino, C₁-C₈-Arylamino, C₁-C₈-Diarylamino, Formyl, Hydroxy, Carboxyl, Cyano, und Halogene wie F, Cl, Br und I substituiert sein können.

Bevorzugte Phosphinliganden sind Triphenylphosphin, Tri(1-naphthyl)phosphin und Tri(o-tolyl)phosphin. Hervorgehoben sind Tri(1-naphthyl)phosphin und Tri(o-tolyl)phosphin.

Alternativ dazu können auch definierte Palladiumkomplexe eingesetzt werden, die aus den oben genannten Liganden in einem oder mehreren Verfahrensschritten zuvor erzeugt wurden.

Beim erfindungsgemäßen Verfahren werden 1 - 20 Moläquivalente Phosphin bezogen auf die eingesetzte Menge Palladium eingesetzt. Vorzugsweise werden 1 - 4 Moläquivalente eingesetzt.

Im erfindungsgemäßen Verfahren wird ein Phasentransferkatalysator aus der Reihe der quaternären Ammoniumsalze, der quaternären Phosphoniumsalze und der Metallsalze, die ihrerseits durch Kronenether oder Kryptanden solubisiert sind, eingesetzt.

Dieser Phasentransferkatalysator besitzt vorzugsweise die allgemeine Formel (IV)

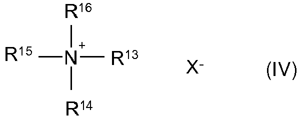

Die Reste R¹³, R¹⁴, R¹⁵ und R¹⁶ stehen unabhängig voneinander gleich oder verschieden für C₁-C₂₈-Alkyl, gegebenenfalls verzweigtes C₁-C₂₈-Alkyl, C₆-C₁₀-Aryl, oder Benzyl.

Der Rest X steht für Halogen, Hydrogensulfat, Sulfat, Dihydrogenphosphat, Hydrogenphosphat, Phosphat oder Acetat.

Bevorzugt steht X für Brom, Chlor, Fluor, Hydrogensulfat, Sulfat, Phosphat und Acetat.

Als solche Phasentransferkatalysatoren seien beispielhaft Tetrabutylammoniumfluorid, -chlorid, - bromid, -iodid, -acetat, Tetraethylammoniumiodid, Benzyltriethylammoniumbromid, Dodecyltrimethylammoniumbromid und Methyl-tridecylammoniumchlorid (Aliquat 336) genannt. Hervorgehoben sind Tetrabutylammoniumfluorid, -acetat, und Benzyltriethylammoniumbromid.

Die Menge an Phasentransferkatalysator im erfindungsgemäßen Verfahren liegt zwischen 1 und 50 Molprozent, bezogen auf Arylboronsäure. Bevorzugt sind Mengen zwischen 5 und 20 Molprozent.

Das erfindungsgemäße Verfahren wird bei Temperaturen von -20 °C bis 200 °C, vorzugsweise bei 0 °C bis 150 °C und besonders bevorzugt bei 20°C bis 120 °C durchgeführt.

Das erfindungsgemäße Verfahren kann in Gegenwart eines Lösungsmittels oder in Substanz durchgeführt werden. Vorzugsweise wird in Gegenwart eines Lösungsmittels gearbeitet. Bevorzugte Lösungsmittel sind gesättigte aliphatische Kohlenwasserstoffe, alicyclische Kohlenwasserstoffe, aromatische Kohlenwasserstoffe, Alkohole, Amide, Sulfoxide, Sulfonate, Nitrile, Ester oder Ether.

Beispielsweise können als Lösungsmittel Pentan, Hexan, Heptan, Octan, Cyclohexan, Toluol, Xylole, Ethylbenzol, Mesitylen, Dioxan, Tetrahydrofuran, Dibutylether, Methyl-t-butylether, Diisopropylether, Diethylenglycol-dimethylether, Methanol, Ethanol, Propanol, Isopropanol, Methylacetat, Ethylacetat, t-Butylacetat, Dimethylformamid, Diethylformamid, N-Methylpyrolidon, Dimethylacetamid, Dimethylsulfoxid, Sulfolan, Acetonitril, Propionitril oder Wasser eingesetzt werden.

Besonders bevorzugt werden aromatische Kohlenwasserstoffe, Amide, Ester und Ether eingesetzt. Ganz besonders bevorzugt werden Ether eingesetzt.

Das erfindungsgemäße Verfahren wird üblicherweise bei Normaldruck durchgeführt, kann aber auch bei verringertem oder erhöhtem Druck durchgeführt werden.

Zur Isolierung der erfindungsgemäß hergestellten Aryl- und Heteroarylessigsäuren und ihrer Derivate wird das Reaktionsgemisch nach Beendigung der Reaktion vorzugsweise destillativ und/oder durch Extraktion aufgearbeitet. Vorzugsweise wird das Reaktionsgemisch durch Extraktion und anschließende Destillation aufgearbeitet.

Das erfindungsgemäße Verfahren wird durch die folgenden Beispiele veranschaulicht, ohne auf diese eingeschränkt zu sein.

### Beispiel 1: 2,6-Dimethylphenylessigsäure-ethylester

In einem 25 mL-Dreihalskolben mit Magnetrührer, Rückflußkühler und Tropftrichter werden 20 mL Tetrahydrofuran (THF), das über Molsieb getrocknet wurde, vorgelegt. Man erhitzt das THF unter Durchleiten von Argon für einige Minuten auf 50°C und kühlt dann wieder auf Raumtemperatur ab. Weiterhin unter Argon werden nun zugegeben: 17.2 mg [0.03 mmol = 0.3 mol%] Bis(dibenzylidenaceton)palladium, 27.4 mg P(o-Tolyl)₃ (Tri-o-tolylphosphin) [0.09 mmol], 2.91 g Kaliumfluorid (getrocknet über P₂O₅ bei 140°C), 272 m g [1 mmol = 10 mol%] Benzyltriethylammoniumbromid, 1.5 g [10 mmol] 2,6-Dimethylphenyl-boronsäure und 2.56 g [15 mmol] Bromessigsäure-ethylester. Dieses Gemisch wird 24 Stunden im leichten Argonstrom unter Rückfluss gerührt. Danach wird über etwas Celite filtriert und der Filterkuchen dreimal mit je 20 mL Essigester gewaschen. Die vereinigten Filtrate werden mit 20 mL 1 N Salzsäure ausgeschüttelt. Die wässrige Phase wird zweimal mit je 20 mL Essigester rückextrahiert. Die vereinigten organischen Phasen werden dann mit 20 mL gesättigter wässriger NaCl-Lösung gewaschen, getrocknet und eingeengt. Man erhält 2.16 g eines Öles, das lt. GC/MS neben 8.9 F1.% m-Xylol 72.9 F1.% 2,6-Dimethylphenylessigsäure-ethylester enthält. Das entspricht einer Ausbeute von 81.9% der Theorie.

### Vergleichsbeispiel 1: 2,6-Dimethylphenylessigsäure-ethylester

In einem 25 mL-Dreihalskolben mit Magnetrührer, Rückflußkühler und Tropftrichter werden 20 mL Tetrahydrofuran (THF), das über Molsieb getrocknet wurde, und 0.36 ml Wasser vorgelegt. Man erhitzt unter Durchleiten von Argon für einige Minuten auf 50°C und kühlt dann wieder auf Raumtemperatur ab. Weiterhin unter Argon werden nun zugegeben: 17.2 mg [0.03 mmol = 0,3 mol%] Bis(dibenzylidenaceton)palladium, 27.4 mg P(o-Tolyl)₃ [0.09 mmol], 2.91 g Kaliumfluorid (getrocknet über P₂O₅ bei 140°C), 1.5 g [10 mmol] 2,6-Dimethylphenyl-boronsäure und 2.56 g [15 mmol] Bromessigsäure-ethylester. Dieses Gemisch wird 24 Stunden im leichten Argonstrom unter Rückfluss gerührt. Danach wird über etwas Celite filtriert und der Filterkuchen dreimal mit je 20 mL Essigester gewaschen. Die vereinigten Filtrate werden mit 20 mL 1 N Salzsäure ausgeschüttelt. Die wässrige Phase wird zweimal mit je 20 mL Essigester rückextrahiert. Die vereinigten organischen Phasen werden dann mit 20 mL gesättigter wässriger NaCl-Lösung gewaschen, getrocknet und eingeengt. Man erhält 1.59 g eines Öles, das lt. GC/MS neben 10.7 F1.% m-Xylol 56.6 F1.% 2,6-Dimethylphenylessigsäure-ethylester enthält. Das entspricht einer Ausbeute von 46.8% der Theorie.

### Beispiele 2 bis 10

### Allgemeine Versuchsbeschreibung:

Die Boronsäure (1.00 mmol), Pd(dba)₂ (Bis(dibenzylidenaceton)palladium) (1.73 mg, 3.00 umol), Tri-o-tolylphosphin (2.74 mg, 9.00 µmol), Benzyltriethylammoniumbromid (27.8 mg, 0.10 mmol) und Kaliumfluorid (174 mg, 3.00 mmol) werden in ein 20 mL Vial unter Luftsauerstoff gegeben. Das Gefäß wird verschlossen, dreimal evakuiert und mit Stickstoff rückbefüllt. Ethylbromacetat (167 mg, 166 µL, 1.50 mmol) und 2 mL THF (trocken, entgast mit Argon) werden unter Rühren hinzugegeben. Die Reaktionsmischung wird bei 60 °C für 24 Stunden gerührt. Nach abgelaufener Reaktionszeit wird abgekühlt, 50 µL *n*-Tetradecan hinzugegeben, eine 0.25 mL Probe entnommen, in 3 mL Ethylessigester und 2 mL Wasser gewaschen, 0.25 mL entnommen, durch eine Pipette mit Celite/ bas. Alox. gefiltert und anschließend per GC analysiert.

Aufarbeitung: Die Reaktionslösung wird über eine Celite / bas. Alox. (1:2) Kombination filtriert. Der Filterkuchen wird mit Ethylessigester gewaschen. Das Filtrat wird eingeengt und mittels Säulenchromatographie (5:1, Hexan / Ethylessigester, Kieselgel) gereinigt. Zurück bleibt das Produkt.

### Beispiel 2: 2,4,6-Trimethylphenylessigsäureethylester

2,4,6-Trimethylphenylessigsäureethylester wurde nach der allgemeinen Versuchsvorschrift aus 2,4,6-Trimethylphenylboronsäure (164 mg, 1.00 mmol) hergestellt. Nach säulenchromatographischer Aufarbeitung (Hexan / Essigester, 5:1) erhält man 2,4,6-Trimethylphenylessigsäureethylester als farblose Flüssigkeit in einer Ausbeute von 62% d.Theorie. ¹H-NMR (200 MHz, CDCl₃): δ= 6.89 (s, 2H), 4.10-4.25 (m, 2H), 3.67 (s, 2H), 2.27-2.37 (m, 9H), 1.27 (t, *J* = 7.1 Hz, 3H) ppm; ¹³C-NMR (50 MHz, CDCl₃): δ= 171.9, 137.4, 136.8, 129.3, 129.2, 61.1, 35.6, 21.3, 20.6, 14.7 ppm; MS (70 eV), *m*/*z* (%): 206 (23) [M⁺], 160, (7), 133 (100), 105 (10) 91 (11); IR (NaCl): υ̃ = 2978 (m), 2867 (w), 1732 (s), 1157 (m), 1031 (m) cm⁻¹.

### Beispiel 3: 4-Acetylphenylessigsäureethylester

4-Acetylphenylessigsäureethylester wurde nach der allgemeinen Versuchsvorschrift aus 4-Acetylphenylboronsäure (164 mg, 1.00 mmol) hergestellt. Nach säulenchromatographischer Aufarbeitung (Hexan / Essigester, 5:1) erhält man 4-Acetylphenylessigsäureethylester als farblosen Feststoff in einer Ausbeute von 60% d. Theorie. ¹H-NMR (400 MHz, CDCl₃): δ= 7.90 (d, *J* = 8.2 Hz, 2H), 7.37 (d, *J* = 8.5 Hz, 2H), 4.11-4.17 (m, 2H), 3.66 (s, 2H), 2.58 (s, 3H), 1.20-1.27 (m, 3H) ppm; ¹³C-NMR (151 MHz, CDCl₃): δ= 197.7, 170.8, 139.5, 136.0, 129.6, 128.6, 61.1, 41.3, 26.6, 14.2 ppm; MS (70 eV), *m*/*z* (%): 192 (100),164 (16), 134 (14), 105 (19), 89 (11); IR (KBr): υ̃ = 2981 (w), 1735 (s), 1682 (m), 1274 (m), 1178 (m) cm⁻¹, Elementar Analyse: (theor): C = 69.89, H = 6.84, (exp): C = 69.95, H = 6.99. Schmelzpunkt: 55-56°C.

### Beispiel 4: 4-Chlor-2,6-dimethylphenylessigsäureethylester

4-Chlor-2,6-dimethylphenylessigsäureethylester wurde nach der allgemeinen Versuchsvorschrift aus 4-Chlor-2,6-dimethylphenylboronsäure (186 mg, 1.00 mmol) hergestellt. Dabei wurden 290 mg [5 mmol] KF und 418 mg [2.5 mmol] Ethylbromacetat eingesetzt. Nach säulenchromatographischer Aufarbeitung (Hexan / Essigester, 5:1) erhält man 4-Chlor-2,6-dimethylphenylessigsäureethylester als farblose Flüssigkeit in einer Ausbeute von 68% d. Theorie.¹H-NMR (400 MHz, CDCl₃): δ= 7.02 (s, 2H), 4.13 (q, *J* = 0 7. Hz, 2H), 3.62 (s, 2H), 2.29 (s, 6H), 1.23 (t, *J* = 7.2 HZ, 3H) ppm; ¹³C-NMR (101 MHz, CDCl₃): δ= 170.8, 139.0, 132.2, 131.6, 130.3, 127.9, 113.6, 60.8, 35.0, 20.1, 14.2 ppm; MS (70 eV), *m*/*z* (%): 226 (22) [M⁺], 180 (9), 153 (100), 115 (17), 91 (11); IR (NaCl): υ̃ = 2980 (m), 1733 (s), 1328 (w), 1155 (m), 1030 (m) cm⁻¹; Elementar Analyse: (theor): C = 63.58, H = 6.67, (exp): C = 63.30, H = 6.78.

### Beispiel 5: 2,6-Diethyl-4-methylphenylessigsäureethylester

2,6-Diethyl-4-methylphenylessigsäureethylester wurde nach der allgemeinen Versuchsvorschrift aus 2,6-Diethyl-4-methylphenylboronsäure (186 mg, 1.00 mmol) hergestellt. Dabei wurden 5.75 mg [0.01 mmol] Pd(dba)₂ und 9.1 mg [0.03 mmol] P(o-Tolyl)₃ eingesetzt. Nach säulenchromatographischer Aufarbeitung (Hexan / Essigester, 5:1) erhält man 2,6-Diethyl-4-methylphenylessigsäureethylester als farblose Flüssigkeit in einer Ausbeute von 54% d. Theorie. ¹H-NMR (400 MHz, CDCl₃): δ= 6.91 (s, 2H), 4.16 (q, *J* = 7.2 Hz, 2H), 3.71 (s, 2H), 2.61-2.69 (m, 4H), 2.32 (s, 3H), 1.19-1.28 (m, 9H) ppm; ¹³C-NMR (101 MHz, CDCl₃): δ= 171.9, 143.0, 136.7, 127.2, 127.1, 60.6, 34.0, 26.4, 21.1, 15.1, 14.2 ppm; MS (70 eV), *m*/*z* (%): 234 (37) [M⁺], 161 (100), 147 (33), 133 (40), 119 (13); IR (NaCl): υ̃ = 2966 (s), 1739 (s), 1458 (w), 1156 (m), 1032 (m) cm⁻¹; Elementar Analyse: (theor): C = 76.88, H = 9.46, (exp): C = 75.85, H = 9.38.

### Beispiel 6: 1-Naphthalinessigsäureethylester

1-Naphthalinessigsäureethylester wurde nach der allgemeinen Versuchsvorschrift aus 1-Naphthalinboronsäure (172 mg, 1.00 mmol) hergestellt. Nach säulenchromatographischer Aufarbeitung (Hexan / Essigester, 5:1) erhält man 1-Naphthalinessigsäureethylester als farblose Flüssigkeit in einer Ausbeute von 77% d. Theorie. ¹H-NMR (600 MHz, CDCl₃): δ= 8.04 (d, *J* = 8.3 Hz, 1H), 7.89 (d, *J* = 8.1 Hz, 1H), 7.80-7.84 (m, 1H), 7.55-7.58 (m, 1H), 7.50-7.54 (m, 1H), 7.43-7.47 (m, 2H), 4.16-4.20 (m, 2H), 4.09 (s, 2H), 1.23-1.27 (m, 3H) ppm; ¹³C-NMR (151 MHz, CDCl₃): δ= 171.7, 133.9, 132.2, 130.8, 128.8, 128.1, 128.0, 126.4, 125.8, 125.6, 123.9, 61.0, 39.4, 14.3 ppm; MS (70 eV), *m*/*z* (%): 214 (100) [M⁺], 141 (34), 115 (45), 89 (9), 63 (6); IR (NaCl): υ̃ = 3047 (w), 2981 (m), 1733 (s), 1173 (m), 1029 (m) cm⁻¹, Elementar Analyse: (theor): C = 78.48, H = 6.59, (exp): C = 78.35, H = 6.86.

### Beispiel 7: 4-Methoxyphenylessigsäureethylester

4-Methoxyphenylessigsäureethylester wurde nach der allgemeinen Versuchsvorschrift aus 4-Methoxyphenylboronsäure (152 mg, 1.00 mmol) hergestellt. Nach säulenchromatographischer Aufarbeitung (Hexan / Essigester, 5:1) erhält man 4-Methoxyphenylessigsäureethylester als farblose Flüssigkeit in einer Ausbeute von 77% d. Theorie. ¹H-NMR (200 MHz, CDCl₃): δ= 7.25 (d, *J* = 8.5 Hz, 2H), 6.91 (d, *J* = 8.7 Hz, 2H), 4.13-4.26 (m, 2H), 3.82 (s, 3H), 3.59 (s, 2H), 1.24-1.26 (m, 3H) ppm; ¹³C-NMR (50 MHz, CDCl₃): δ= 172.3, 159.1, 130.7, 126.7, 114.4, 61.1, 55.6, 40.9, 14.6 ppm; MS (70 eV), *m*/*z* (%): 194 (24) [M⁺], 121 (100), 91 (8), 77 (10), 51 (4); IR (NaCl): υ̃ = 2981 (m), 2836 (w), 1732 (s), 1513 (s), 1247 (m), 1032 (m) cm⁻¹; Elementar Analyse: (theor): C = 68.02, H = 7.27, (exp): C = 67.91, H = 7.18.

### Beispiel 8: 4-Ethoxycarbonyl-phenylessigsäureethylester

1,4-Phenyldiessigsäure-1,4-diethylester wurde nach der allgemeinen Versuchsvorschrift aus 4-Ethoxycarbonylphenylboronsäure (194 mg, 1.00 mmol) hergestellt. Nach säulenchromatographischer Aufarbeitung (Hexan / Essigester, 5:1) erhält man 1,4-Phenyldiessigsäure-1,4-diethylester als gelbe Flüssigkeit in einer Ausbeute von 71% d. Theorie. ¹H-NMR (600 MHz, CDCl₃): δ= 8.12 (d, *J* = 8.6 Hz, 1H), 7.99 (d, *J* = 8.3 Hz, 1H), 7.67 (d, *J* = 8.3 Hz, 1H), 7.34 (d, *J* = 8.1 Hz, 1H), 4.38-4.42 (m, 1H), 4.34-4.37 (m, 1H), 4.12-4.16 (m, 1H), 3.65 (s, 1H), 1.36-1.42 (m, 4H), 1.22-1.26 (m, 2H) ppm; ¹³C-NMR (151 MHz, CDCl₃): δ= 170.9, 166.4, 139.2, 130.2, 129.8, 129.4, 129.3, 127.2, 61.1, 61.0, 41.4, 14.4, 14.2 ppm; MS (70 eV), *m*/*z* (%): 237 (6) [M⁺], 191 (39), 163 (100), 135 (39), 118 (13); IR (NaCl): υ̃ = 2982 (m), 2938 (w), 1735 (s), 1718 (s) 1277 (s) cm⁻¹; Elementar Analyse: (theor): C = 66.09, H = 6.83, (exp): C = 65.98, H = 7.05.

### Beispiel 9: 2,6-Dimethylphenylessigsäure-ethylester

2,6-Dimethylphenylessigsäure-ethylester wurde nach der allgemeinen Vorschrift unter Verwendung von 0.1 mmol Tetrabutylammoniumfluorid anstelle von Benzyltriethylammoniumbromid in einer Ausbeute von 63% d. Th. hergestellt.

### Beispiel 10: 2,6-Dimethylphenylessigsäure-ethylester

2,6-Dimethylphenylessigsäure-ethylester wurde nach der allgemeinen Vorschrift unter Verwendung von 0.1 mmol Tetrabutylammoniumacetat anstelle von Benzyltriethylammoniumbromid in einer Ausbeute von 66% d. Th. hergestellt

### Beispiel 11: ω-(2,6-Dimethylphenyl)acetophenon

2,6-Dimethylphenylboronsäure (151 mg, 1.01 mmol), ω-Bromacetophenon (299 mg, 1.50 mmol), Pd(dba)₂ (5.75 mg, 10.0 µmol), Tri-o-tolylphosphin (10.0 mg, 32.9 µmol), Benzyltriethylammoniumbromid (27.2 mg, 0.10 mmol) und Kaliumfluorid (323 mg, 5.56 mmol) werden in ein 20 mL Vial unter Luftsauerstoff gegeben. Das Gefäß wird verschlossen, dreimal evakuiert und mit Stickstoff rückbefüllt. 2 mL THF (trocken, entgast mit Argon) werden unter Rühren hinzugegeben. Die Reaktionsmischung wird bei 60 °C für 24 Stunden gerührt. Nach abgelaufener Reaktionszeit wird abgekühlt, 50 µL n-Tetradecan hinzugegeben, eine 0.25 mL Probe entnommen, in 3 mL Ethylessigester und 2 mL Wasser gewaschen, 0.25 mL entnommen, durch eine Pipette mit Celite/ bas. Alox. gefiltert und anschließend per GC analysiert.

Aufarbeitung: Die Reaktionslösung wird über eine Celite / bas. Alox. (1:2) Kombination filtriert. Der Filterkuchen wird mit Ethylessigester gewaschen. Das Filtrat wird eingeengt und mittels Säulenchromatographie (5:1, Hexan / Ethylessigester, Kieselgel) gereinigt. Zurück bleiben : ω-(2,6-Dimethylphenyl)acetophenon (106 mg, 0.473 mmol, 47% d.Th.).

¹H-NMR (400 MHz, CDCl₃): δ= 8.16 (d, *J* = 7.2 Hz, 2H), 7.66 (d, *J* = 7.4 Hz, 1H), 7.51-7.61 (m, 2H), 7.12-7.21 (m, 3H), 4.45 (s, 2H), 2.30 (s, 6H) ppm; ¹³C-NMR (101 MHz, CDCl₃): δ= 196.9, 137.4, 137.0, 133.1, 132.5, 128.7, 128.1, 128.0, 126.9, 39.7 ppm; MS (70 eV), *m*/*z* (%): 224 (11) [M⁺], 119 (9), 106 (8), 105 (100), 91 (9), 77 (33), 51 (11); Elementaranalyse: (theor): C = 85.68, H = 7.19, (exp): C = 85.39, H = 7.22; Schmelzpunkt: 113-114°C.

### Beispiel 12: N-(Phenylacetyl)piperidin

Benzolboronsäure (122 mg, 1.00 mmol), Pd(dba)₂ (1.73 mg, 3.0 µmol), Tri-*1*-naphthylphosphin (3.71 mg, 9.00 µmol), Benzyltriethylammoniumbromid (27.2 mg, 0.10 mmol) und Kaliumfluorid (290 mg, 5.00 mmol) werden in ein 20 mL Vial unter Luftsauerstoff gegeben. Das Gefäß wird verschlossen, dreimal evakuiert und mit Stickstoff rückbefüllt. N-(Bromacetyl)-piperidin (309 mg, 1.50 mmol) und 2 mL THF (trocken, entgast mit Argon) werden unter Rühren hinzugegeben. Die Reaktionsmischung wird bei 60 °C für 24 Stunden gerührt. Nach abgelaufener Reaktionszeit wird abgekühlt, 50 µL n-Tetradecan hinzugegeben, eine 0.25 mL Probe entnommen, in 3 mL Ethylessigester und 2 mL Wasser gewaschen, 0.25 mL entnommen, durch eine Pipette mit Celite/ bas. Alox. gefiltert und anschließend per GC analysiert.

Aufarbeitung: Die Reaktionslösung wird über eine Celite / bas. Alox. (1:2) Kombination filtriert. Der Filterkuchen wird mit Ethylessigester gewaschen. Das Filtrat wird eingeengt und mittels Säulenchromatographie (1:2, Hexan / Ethylessigester, Kieselgel) gereinigt. Zurück bleiben N-Phenylacetylpiperidin (169 mg, 0.714 mmol, 71% d.Th.).

¹H-NMR (600 MHz, CDCl₃): δ= 7.29 (t, *J* = 7.5 Hz, 2H), 7.19-7.24 (m, 2H), 3.71 (s, 2H), 3.52-3.56 (m, 2H), 3.33-3.37 (m, 2H), 1.60-1.65 (m, 1H), 1.52-1.57 (m, 2H), 1.47-1.52 (m, 2H), 1.30-1.34 (m, 2H) ppm; ¹³C-NMR (151 MHz, CDCl₃): δ= 169.3, 135.5, 128.7, 126.6, 48.0, 47.3, 43.3, 42.9, 41.2, 31.0, 26.2, 25.5, 25.4, 24.3 ppm; MS (70 eV), *m*/*z* (%): 203 (44) [M⁺], 112 (100), 91 (41), 84 (14), 69 (57), 65 (18), 41 (29).

## Patentansprüche

1. Verfahren zur Herstellung von Verbindungen der Formel (III) 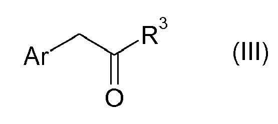 in welcher
Ar für die Gruppe 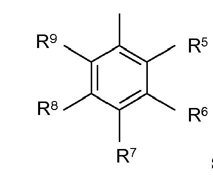 steht oder
Ar für einen heteroaromatischen Rest wie 2-Pyridyl, 3-Pyridyl, 4-Pyridyl, 2-Furyl-, 3-Furyl, 2-Thienyl, oder 3-Thienyl steht oder
Ar für 1- oder 2-Naphthyl steht,
wobei R⁵, R⁶, R⁷, R⁸ und R⁹ unabhängig voneinander gleich oder verschieden für Wasserstoff, Halogen, für gegebenenfalls durch Halogen substituiertes C₁-C₆-Alkyl, C₁-C₆-Alkoxy, Phenyl, -CO-C₁-C₃-Alkyl, -COO-C₁-C₆-Alkyl oder -COO-C₆-C₁₀-Aryl stehen,
R³ für Hydroxy, für jeweils gegebenenfalls substituiertes C₁-C₈-Alkyl, C₁-C₈-Alkoxy, Phenyl, Aryl, Phenoxy oder Aryloxy oder für NR⁴R^{4'} steht,
wobei R⁴ und R^{4'} unabhängig voneinander gleich oder verschieden für Wasserstoff, C₁-C₄-Alkyl oder für gegebenenfalls durch C₁-C₃-Alkyl, welches durch Fluor oder Chlor substituiert sein kann, durch Nitro, Cyano oder Di-C₁-C₃-Alkylamino substituiertes Phenyl stehen, oder gemeinsam mit dem Stickstoffatom an das sie gebunden sind für einen gesättigten oder ungesättigten, substituierten oder unsubstituierten Cyclus stehen,
**dadurch gekennzeichnet, dass** man Verbindungen der Formel (I) 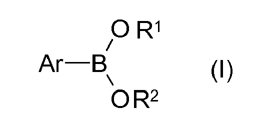 in welcher
R¹ für Wasserstoff oder C₁-C₈-Alkyl steht,
R² für Wasserstoff oder C₁-C₈-Alkyl steht, oder
R¹ und R² gemeinsam mit den Atomen, an die sie gebunden sind für einen gesättigten oder ungesättigten, substituierten oder unsubstituierten Cyclus stehen,
und Ar die oben angegebenen Bedeutungen hat
mit Verbindungen der Formel (II) 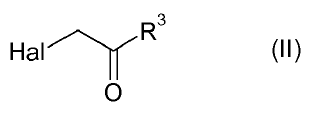 in welcher
Hal für Halogen steht,
und R³ die oben angegebenen Bedeutungen hat
in Gegenwart eines Palladiumkatalysators, eines Phosphin-Liganden, einer anorganischen Base, und eines Phasentransferkatalysators gegebenenfalls unter Verwendung eines organischen Lösungsmittels umsetzt.

2. Verfahren zur Herstellung von Verbindungen der Formel (III) gemäß Anspruch 1 **dadurch gekennzeichnet, dass**
R¹ für Wasserstoff oder C₁-C₄-Alkyl steht,
R² für Wasserstoff oder C₁-C₄-Alkyl steht, oder
R¹ und R² gemeinsam mit den Atomen, an die sie gebunden sind für gegebenenfalls durch C₁-C₄-Alkyl oder Aryl substituiertes C₂-C₃-Alkandiyl stehen,
R³ für Hydroxy, gegebenenfalls durch Fluor substituiertes C₁-C₄-Alkyl, C₁-C₄-Alkoxy, für jeweils gegebenenfalls substituiertes Phenyl, Phenoxy oder für NR⁴R^{4'} steht,
wobei R⁴ und R^{4'} unabhängig voneinander gleich oder verschieden für Wasserstoff, Methyl, Ethyl, i-Propyl, n-Propyl oder für gegebenenfalls durch Methyl, Ethyl, i-Propyl, n-Propyl, CF₃, C₂F₅, C₃F₇, Nitro, Cyano, N (Methyl)₂, N(Ethyl)₂, N(n-Propyl)₂, N(i-Propyl)₂ substituiertes Phenyl stehen, oder gemeinsam mit dem Stickstoffatom an das sie gebunden sind für einen gesättigten oder ungesättigten, substituierten oder unsubstituierten 5- oder 6-Cyclus stehen,
Ar für 1- oder 2-Naphthyl oder für die Gruppe 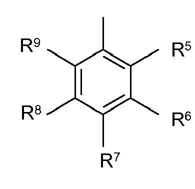 steht, wobei
R⁵, R⁶, R⁷, R⁸ und R⁹ unabhängig voneinander gleich oder verschieden für Wasserstoff, Fluor, Chlor, für gegebenenfalls durch Fluor substituiertes C₁-C₄-Alkyl, C₁-C₄-Alkoxy, Phenyl, -CO-C₁-C₃-Alkyl, -COO-C₁-C₄-Alkyl oder -COO-C₆-C₈-Aryl stehen,
Hal für Fluor, Chlor, Brom oder Iod steht.

3. Verfahren zur Herstellung von Verbindungen der Formel (III) gemäß Anspruch 1 **dadurch gekennzeichnet, dass**
R¹ für Wasserstoff, Methyl, Ethyl, i-Propyl oder n-Propyl steht,
R² für Wasserstoff, Methyl, Ethyl, i-Propyl oder n-Propyl, oder
R¹ und R² gemeinsam mit den Atomen, an die sie gebunden sind für gegebenenfalls einfach bis vierfach durch Methyl substituiertes C₂-Alkandiyl, für gegebenenfalls einfach bis sechsfach durch Methyl substituiertes C₃-Alkandiyl stehen,
R³ für Methyl, Ethyl, i-Propyl, n-Propyl, CF₃, C₂F₅, C₃F₇, Methoxy, Ethoxy, i-Propoxy, n-Propoxy oder tert.-Butoxy, für jeweils gegebenenfalls substituiertes Phenyl oder für NR⁴R^{4'} steht,
wobei R⁴ und R^{4'} unabhängig voneinander gleich oder verschieden für Wasserstoff, Methyl, Ethyl, i-Propyl, n-Propyl oder gemeinsam mit dem Stickstoffatom an das sie gebunden sind für einen gesättigten, unsubstituierten 5- oder 6-Cyclus stehen,
Ar für 1-Naphthyl oder für die Gruppe 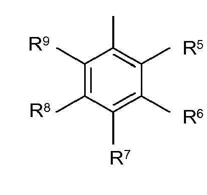 steht, wobei
R⁵, R⁶, R⁷, R⁸ und R⁹ unabhängig voneinander gleich oder verschieden für Wasserstoff, Fluor, Chlor, Methyl, Ethyl, , i-Propyl, n-Propyl CF₃, C₂F₅, C₃F₇, Methoxy, Ethoxy, Phenyl, -CO-Methyl, -CO-Ethyl, -COO-Methyl, -COO-Ethyl oder -COO-Phenyl stehen,
Hal für Chlor, Brom oder Iod steht.

4. Verfahren zur Herstellung von Verbindungen der Formel (III) gemäß Anspruch 1 **dadurch gekennzeichnet, dass**
R¹ für Wasserstoff steht,
R² für Wasserstoff steht,
R³ für Methoxy, Ethoxy, tert.-Butoxy, Phenyl oder für NR⁴R^{4'} steht,
wobei R⁴ und R^{4'} gemeinsam mit dem Stickstoffatom, an das sie gebunden sind für einen gesättigten, unsubstituierten 6-Cyclus stehen,
Ar für 1-Naphthyl, Phenyl, 2,6-Dimethylphenyl, 2,4,6-Trimethylphenyl, 4-Acetylphenyl, 4-Chlor-2,6-dimethylphenyl, 2,6-Diethyl-4-methylphenyl, 4-Methoxyphenyl, 4-Ethoxycarbonylphenyl steht,
Hal für Brom steht.

5. Verfahren zur Herstellung von Verbindungen der Formel (III) gemäß Anspruch 1 **dadurch gekennzeichnet, dass** als Palladiumkatalysatoren Bis(dibenzylidenaceton)palladium, Tris(dibenzylidenaceton)dipalladium, Palladiumchlorid, Palladiumbromid oder Palladiumacetat eingesetzt werden.

6. Verfahren zur Herstellung von Verbindungen der Formel (III), gemäß Anspruch 1 **dadurch gekennzeichnet, dass** als Phosphin-Liganden Triphenylphosphin, Tri(1-naphthyl)phosphin oder Tri(o-tolyl)phosphin eingesetzt werden.

7. Verfahren zur Herstellung von Verbindungen der Formel (III), gemäß Anspruch 1 **dadurch gekennzeichnet, dass** als Basen Kaliumfluorid, Kaliumcarbonat oder Kaliumphosphat eingesetzt werden.

8. Verfahren zur Herstellung von Verbindungen der Formel (III), gemäß Anspruch 1, **dadurch gekennzeichnet, dass** als Phasentransferkatalysatoren Tetrabutylammoniumfluorid, -chlorid, - bromid, -iodid, -acetat, Tetraethylammoniumiodid, Benzyltriethylammoniumbromid, Dodecyltrimethylammoniumbromid oder Methyl-tridecylammoniumchlorid eingesetzt werden.

## Claims

1. Process for preparing compounds of the formula (III) 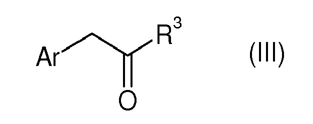 in which
Ar is the group 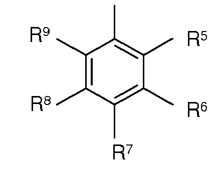 or
Ar is a heteroaromatic radical such as 2-pyridyl, 3-pyridyl, 4-pyridyl, 2-furyl, 3-furyl, 2-thienyl or 3-thienyl, or
Ar is 1- or 2-naphthyl,
where R⁵, R⁶, R⁷, R⁸ and R⁹ are the same or different and are each independently hydrogen, halogen, optionally halogen-substituted C₁-C₆-alkyl, C₁-C₆-alkoxy, phenyl, -CO-C₁-C₃-alkyl, -COO-C₁-C₆-alkyl or -COO-C₆-C₁₀-aryl,
R³ is hydroxyl, in each case optionally substituted C₁-C₈-alkyl, C₁-C₈-alkoxy, phenyl, aryl, phenoxy or aryloxy, or NR⁴R^{4'},
where R⁴ and R^{4'} are the same or different and are each independently hydrogen, C₁-C₄-alkyl, or phenyl optionally substituted by C₁-C₃-alkyl which may be substituted by fluorine or chlorine, or by nitro, cyano or di-C₁-C₃-alkylamino, or, together with the nitrogen atom to which they are bonded, are a saturated or unsaturated, substituted or unsubstituted cycle, **characterized in that**
compounds of the formula (I) 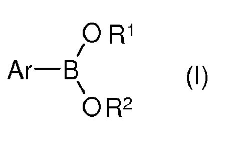 in which
R¹ is hydrogen or C₁-C₈-alkyl,
R² is hydrogen or C₁-C₈-alkyl, or
R¹ and R² together with the atoms to which they are bonded are a saturated or unsaturated, substituted or unsubstituted cycle,
and Ar is as defined above
are reacted with compounds of the formula (II) 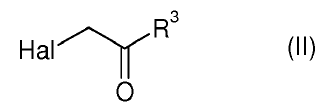 in which
Hal is halogen,
and R³ is as defined above,
in the presence of a palladium catalyst, of a phosphine ligand, of an inorganic base, and of a phase transfer catalyst, optionally using an organic solvent.

2. Process for preparing compounds of the formula (III) according to Claim 1, **characterized in that**
R¹ is hydrogen or C₁-C₄-alkyl,
R² is hydrogen or C₁-C₄-alkyl, or
R¹ and R², together with the atoms to which they are bonded, are optionally C₁-C₄-alkyl- or aryl-substituted C₂-C₃-alkanediyl,
R³ is hydroxyl, optionally fluorine-substituted C₁-C₄-alkyl, C₁-C₄-alkoxy, in each case optionally substituted phenyl, phenoxy, or NR⁴R^{4'},
where R⁴ and R^{4'} are the same or different and are each independently hydrogen, methyl, ethyl, i-propyl, n-propyl, or optionally methyl-, ethyl-, i-propyl-, n-propyl-, CF₃-, C₂F₅-, C₃F₇-, nitro-, cyano-, N(methyl)₂-, N(ethyl)₂-, N(n-propyl)₂-, N(i-propyl)₂-substituted phenyl, or, together with the nitrogen atom to which they are bonded, are a saturated or unsaturated, substituted or unsubstituted, 5- or 6-membered cycle,
Ar is 1- or 2-naphthyl or the group 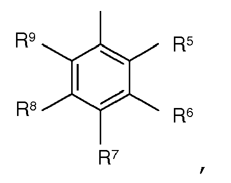 where
R⁵, R⁶, R⁷, R⁸ and R⁹ are the same or different and are each independently hydrogen, fluorine, chlorine, optionally fluorine-substituted C₁-C₄-alkyl, C₁-C₄-alkoxy, phenyl, -CO-C₁-C₃-alkyl, -COO-C₁-C₄-alkyl or -COO-C₆-C₈-aryl,
Hal is fluorine, chlorine, bromine or iodine.

3. Process for preparing compounds of the formula (III) according to Claim 1, **characterized in that**
R¹ is hydrogen, methyl, ethyl, i-propyl or n-propyl,
R² is hydrogen, methyl, ethyl, i-propyl or n-propyl, or R¹ and R² together with the atoms to which they are bonded are optionally mono- to tetra-methyl-substituted C₂-alkanediyl, optionally mono- to hexa-methyl-substituted C₃-alkanediyl,
R³ is methyl, ethyl, i-propyl, n-propyl, CF₃, C₂F₅, C₃F₇, methoxy, ethoxy, i-propoxy, n-propoxy or tert-butoxy, in each case optionally substituted phenyl, or NR⁴R^{4'}, where R⁴ and R^{4'} are the same or different and are each independently hydrogen, methyl, ethyl, i-propyl, n-propyl or, together with the nitrogen atom to which they are bonded, are a saturated, unsubstituted, 5- or 6-membered cycle,
Ar is 1-naphthyl or the group 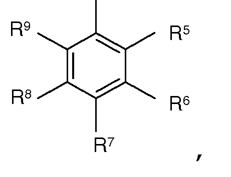 where
R⁵, R⁶, R⁷, R⁸ and R⁹ are the same or different and are each independently hydrogen, fluorine, chlorine, methyl, ethyl, i-propyl, n-propyl, CF₃, C₂F₅, C₃F₇, methoxy, ethoxy, phenyl, -CO-methyl, -CO-ethyl, -COO-methyl, -COO-ethyl or -COO-phenyl,
Hal is chlorine, bromine or iodine.

4. Process for preparing compounds of the formula (III) according to Claim 1, **characterized in that**
R¹ is hydrogen,
R² is hydrogen,
R³ is methoxy, ethoxy, tert-butoxy, phenyl or NR⁴R^{4'}, where R⁴ and R^{4'}, together with the nitrogen atom to which they are bonded, are a saturated, unsubstituted 6-membered cycle,
Ar is 1-naphthyl, phenyl, 2,6-dimethylphenyl, 2,4,6-trimethylphenyl, 4-acetylphenyl, 4-chloro-2,6-dimethylphenyl, 2,6-diethyl-4-methylphenyl, 4-methoxy-phenyl, 4-ethoxycarbonylphenyl,
Hal is bromine.

5. Process for preparing compounds of the formula (III) according to Claim 1, **characterized in that** the palladium catalysts used are bis(dibenzylidene-acetone)palladium, tris(dibenzylidene-acetone)dipalladium, palladium chloride, palladium bromide or palladium acetate.

6. Process for preparing compounds of the formula (III) according to Claim 1, **characterized in that** the phosphine ligands used are triphenylphosphine, tri(1-naphthyl)phosphine or tri(o-tolyl)phosphine.

7. Process for preparing compounds of the formula (III) according to Claim 1, **characterized in that** the bases used are potassium fluoride, potassium carbonate or potassium phosphate.

8. Process for preparing compounds of the formula (III) according to Claim 1, **characterized in that** the phase transfer catalysts used are tetrabutylammonium fluoride, chloride, bromide, iodide or acetate, tetraethylammonium iodide, benzyltriethylammonium bromide, dodecyltrimethylammonium bromide or methyl-tridecylammonium chloride.

## Revendications

1. Procédé de fabrication de composés de formule (III) 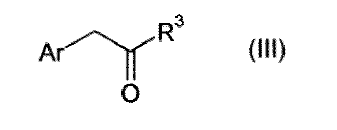 dans laquelle
Ar représente le groupe 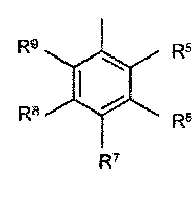 ou
Ar représente un radical hétéroaromatique tel que 2-pyridyle, 3-pyridyle, 4-pyridyle, 2-furyle, 3-furyle, 2-thiényle ou 3-thiényle, ou
Ar représente 1- ou 2-naphtyle,
R⁵, R⁶, R⁷, R⁸ et R⁹ étant indépendamment les uns des autres identiques ou différents et représentant hydrogène, halogène, alkyle en C₁-C₆ éventuellement substitué par halogène, alcoxy en C₁-C₆, phényle, -CO-alkyle en C₁-C₃, -COO-alkyle en C₁-C₆ ou -COO-aryle en C₆-C₁₀,
R³ représente hydroxy, alkyle en C₁-C₈, alcoxy en C₁-C₈, phényle, aryle, phénoxy ou aryloxy, chacun éventuellement substitué, ou NR⁴R^{4'},
R⁴ et R^{4'} étant indépendamment l'un de l'autre identiques ou différents et représentant hydrogène, alkyle en C₁-C₄ ou phényle éventuellement substitué par alkyle en C₁-C₃, qui peut être substitué par fluor ou chlore, par nitro, cyano ou di-alkylamino en C₁-C₃, ou représentant ensemble avec l'atome d'azote auquel ils sont reliés un cycle saturé ou insaturé, substitué ou non substitué,
**caractérisé en ce que**
des composés de formule (I) 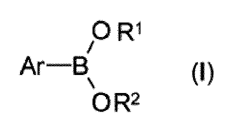 dans laquelle
R¹ représente hydrogène ou alkyle en C₁-C₈,
R² représente hydrogène ou alkyle en C₁-C₈, ou
R¹ et R² représentent ensemble avec les atomes auxquels ils sont reliés un cycle saturé ou insaturé, substitué ou non substitué,
et Ar a les significations indiquées précédemment,
sont mis en réaction avec des composés de formule (II) 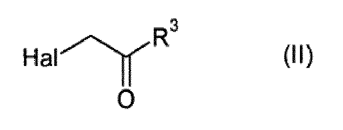 dans laquelle
Hal représente halogène,
et R³ a les significations indiquées précédemment,
en présence d'un catalyseur de palladium, d'un ligand phosphine, d'une base inorganique et d'un catalyseur de transfert de phase, éventuellement en utilisant un solvant organique.

2. Procédé de fabrication de composés de formule (III) selon la revendication 1, **caractérisé en ce que**
R¹ représente hydrogène ou alkyle en C₁-C₄,
R² représente hydrogène ou alkyle en C₁-C₄, ou
R¹ et R² représentent ensemble avec les atomes auxquels ils sont reliés un alcanediyle en C₂-C₃ éventuellement substitué par alkyle en C₁-C₄ ou aryle,
R³ représente hydroxy, alkyle en C₁-C₄ éventuellement substitué par fluor, alcoxy en C₁-C₄ ; phényle, phénoxy, chacun éventuellement substitué ; ou NR⁴R^{4'},
R⁴ et R^{4'} étant indépendamment l'un de l'autre identiques ou différents et représentant hydrogène, méthyle, éthyle, i-propyle, n-propyle ou phényle éventuellement substitué par méthyle, éthyle, i-propyle, n-propyle, CF₃, C₂F₅, C₃F₇, nitro, cyano, N(méthyle)₂, N(éthyle)2, N(n-propyle)₂, N(i-propyle)₂, ou représentant ensemble avec l'atome d'azote auquel ils sont reliés un cycle à 5 ou 6 éléments, saturé ou insaturé, substitué ou non substitué,
Ar représente 1- ou 2-naphtyle ou le groupe 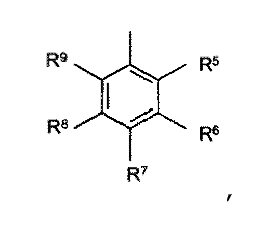 R⁵, R⁶, R⁷, R⁸ et R⁹ étant indépendamment les uns des autres identiques ou différents et représentant hydrogène, fluor, chlore, alkyle en C₁-C₄ éventuellement substitué par fluor, alcoxy en C₁-C₄, phényle, -CO-alkyle en C₁-C₃, -COO-alkyle en C₁-C₄ ou -COO-aryle en C₆-C₈,
Hal représente fluor, chlore, brome ou iode.

3. Procédé de fabrication de composés de formule (III) selon la revendication 1, **caractérisé en ce que**
R¹ représente hydrogène, méthyle, éthyle, i-propyle ou
n-propyle,
R² représente hydrogène, méthyle, éthyle, i-propyle ou n-propyle, ou
R¹ et R² représentent ensemble avec les atomes auxquels ils sont reliés un alcanediyle en C₂ éventuellement substitué une à quatre fois par méthyle, ou alcanediyle en C₃ éventuellement substitué une à six fois par méthyle,
R³ représente méthyle, éthyle, i-propyle, n-propyle, CF₃, C₂F₅, C₃F₇, méthoxy, éthoxy, i-propoxy, n-propoxy ou tert.-butoxy, phényle éventuellement substitué ou NR⁴R^{4'},
R⁴ et R^{4'} étant indépendamment l'un de l'autre identiques ou différents et représentant hydrogène, méthyle, éthyle, i-propyle, n-propyle, ou représentant ensemble avec l'atome d'azote auquel ils sont reliés un cycle à 5 ou 6 éléments, saturé, non substitué,
Ar représente 1-naphtyle ou le groupe 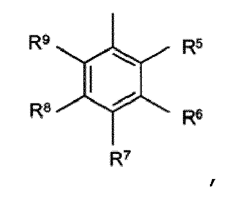 R⁵, R⁶, R⁷, R⁸ et R⁹ étant indépendamment les uns des autres identiques ou différents et représentant hydrogène, fluor, chlore, méthyle, éthyle, i-propyle, n-propyle, CF₃, C₂F₅, C₃F₇, méthoxy, éthoxy, phényle, - CO-méthyle, -CO-éthyle, -COO-méthyle, -COO-éthyle ou - COO-phényle,
Hal représente chlore, brome ou iode.

4. Procédé de fabrication de composés de formule (III) selon la revendication 1, **caractérisé en ce que**
R¹ représente hydrogène,
R² représente hydrogène,
R³ représente méthoxy, éthoxy, tert.-butoxy, phényle ou
NR⁴R^{4'},
R⁴ et R^{4'} représentant ensemble avec l'atome d'azote auquel ils sont reliés un cycle à 6 éléments, saturé, non substitué,
Ar représente 1-naphtyle, phényle, 2,6-diméthylphényle, 2,4,6-triméthylphényle, 4-acétylphényle, 4-chloro-2,6-diméthylphényle, 2,6-diéthyl-4-méthylphényle, 4-méthoxyphényle, 4-éthoxycarbonylphényle,
Hal représente brome.

5. Procédé de fabrication de composés de formule (III) selon la revendication 1, **caractérisé en ce que** le bis(dibenzylidène-acétone)palladium, le tris(dibenzylidène-acétone)dipalladium, le chlorure de palladium, le bromure de palladium ou l'acétate de palladium est utilisé en tant que catalyseur de palladium.

6. Procédé de fabrication de composés de formule (III) selon la revendication 1, **caractérisé en ce que** le triphénylphosphine, la tri(1-naphtyl)phosphine ou la tri(o-tolyl)phosphine est utilisée en tant que ligand phosphine.

7. Procédé de fabrication de composés de formule (III) selon la revendication 1, **caractérisé en ce que** le fluorure de potassium, le carbonate de potassium ou le phosphate de potassium est utilisé en tant que base.

8. Procédé de fabrication de composés de formule (III) selon la revendication 1, **caractérisé en ce que** le fluorure, chlorure, bromure, iodure, acétate de tétrabutylammonium, l'iodure de tétraéthylammonium, le bromure de benzyltriéthylammonium, le bromure de dodécyltriméthylammonium ou le chlorure de méthyl-tridécylammonium est utilisé en tant que catalyseur de transfert de phase.
